# EUROPEAN PATENT SPECIFICATION

(11) **EP 3 906 966 B1**
(45) Date of publication and mention of the grant of the patent: **26.06.2024**
(21) Application number: 21165963.6
(22) Date of filing: 30.03.2021
(51) Int. Cl.: A61N 1/39, A61H 31/00, G16H 40/67, A61B 5/318, G16H 20/30, G16H 50/20

(54) **RESUSCITATION SYSTEM**
REANIMATIONSSYSTEM
SYSTÈME DE RÉANIMATION

(30) Priority: 05.05.2020 GB 202006613; 24.12.2020 GB 202020616
(43) Date of publication of application: 10.11.2021
(73) Proprietor: Stryker European Operations Limited, Carrigtwohill, Co. Cork T45 HX08 (IE)
(72) Inventor: Donaghy, Dymphna, Belfast, BT3 9ED (GB); Anderson, Johnny, Belfast, BT3 9ED (GB); Harvey, Adam, Belfast, BT3 9ED (GB); Lindsay, Peter, Belfast, BT3 9ED (GB)
(74) Representative: HGF

(56) References cited:
- WO-A2-2017/055953
- US-A1- 2018 040 255
- US-A1- 2019 255 340
- US-A1- 2019 365 333
- US-B1- 6 356 785

## Description

The invention relates to a resuscitation system which uses information about a subject to determine a resuscitation strategy and adapt resuscitation treatment for the subject.

Both the European Resuscitation Council and the American Heart Association Guidelines for adult basic life support in a cardiac arrest event, suggest that a rescuer should perform the exact same actions on adult subjects, regardless of age, gender, health status, duration of cardiac arrest etc. However, it has been realised that it is possible that these characteristics and others could influence the condition of the subject and therefore the optimal actions a rescuer should take to achieve successful resuscitation and prevent loss of life.

US2018/0040255 discloses a feedback device for an acute care provider which includes at least one motion sensor, a haptic output component for providing feedback having a varying haptic pattern to the acute care provider regarding performance of a resuscitation activity and a controller. The controller can be configured to receive and process a signal representative of performance of the resuscitation activity from the at least one motion sensor, compare the acute care provider's performance of the resuscitation activity to a target performance of the resuscitation activity, and cause the haptic output component to provide haptic feedback to the acute care provider by changing the haptic pattern based, at least in part, on the signal from the at least one motion sensor and the comparison of the acute care provider's performance to the target performance of the resuscitation activity. The device can be adapted to be wrist-worn by the acute care provider.

US2019/0255340 discloses a medical apparatus which provides resuscitative therapy to a patient. The apparatus includes an electrocardiogram (ECG) input, a defibrillation output configured to provide an electrical defibrillation shock treatment, and an applicator body configured to provide active compression and decompression therapy to the patient's chest. The applicator body includes a rescuer end configured for hands of the rescuer to press and pull on the applicator body, a coupling surface configured to adhere to the patient's chest to provide active compression decompression therapy, a capacitor, and processor(s) configured to receive and analyze the ECG signal of the patient, determine whether the patient is in need of defibrillation, and administer the defibrillation shock treatment to the patient.

US6356785 relates generally to defibrillators and, in particular, to a defibrillator with a built in CPR ("CPR") prompt system. The invention also relates generally to a defibrillator with a built-in Advanced Cardiac Life Support ("ACLS") prompt system. Defibrillators include manual defibrillator, automatic or semi-automatic external defibrillators ("AEDs") and defibrillator trainers. More specifically, this invention is directed to a defibrillator system capable of generating audible prompts and/or visual prompts through an audible prompt generator and a visual prompt generator, wherein the defibrillator instructs a rescuer on performing CPR or ACLS. The instructions are controlled through a prompt generator and may be audible prompts or visual images, or a combination of the two. The prompts may be emitted at a predetermined rate and may be synchronized. This invention is also directed to a method for administering care comprising, providing instructions to a rescuer. The instructions are either CPR instructions, ACLS instructions, or a combination of the two. Both the ACLS and CPR systems are capable of providing instructions for treating both adults and pediatric patients.

WO2017/055953 discloses a portable medical apparatus for real-time individualized treatment guidance for a subject comprising a user interface, a communications module, and a controller. The user interface obtains the subject's identification information. The communications module communicates with at least one of (i) a remote server having stored thereon a cloud-based electronic health record that comprises at least the subject's physical body measurements and (ii) a smartphone located within a given immediate proximity of the portable medical apparatus. The communications module further retrieves, in response to the subject's identification information, the cloud-based electronic health record. The controller generates, in response to the subject's physical body measurements from the cloud-based electronic health record, real-time individualized treatment guidance to be followed by a user of the portable medical apparatus for administering the individualized treatment to the subject.

According to the invention there is provided a resuscitation system for use in the resuscitation of a subject, comprising:
a computing device configured to input subject information and use the subject information to create a subject resuscitation strategy,
a sensor configured to detect subject biosignals and use the subject biosignals to monitor for a subject cardiac arrest event, and
a defibrillator configured to determine and deliver a subject resuscitation treatment, wherein
on determination of a subject cardiac arrest event, the sensor is configured to output sensor subject biosignals to the computing device,
the computing device is configured to use the sensor subject biosignals to adapt the subject resuscitation strategy and output the subject resuscitation strategy to the defibrillator,
the defibrillator is configured to measure subject biodata, use the subject biodata and the subject resuscitation strategy to determine and deliver a subject resuscitation treatment, and
during subject resuscitation treatment,
the defibrillator is configured to repeatedly measure subject biodata and output defibrillator subject biodata to the computing device,
the computing device is configured to repeatedly use the defibrillator subject biodata to adapt the subject resuscitation strategy and output the subject resuscitation strategy to the defibrillator,
the defibrillator is configured to repeatedly use the measured subject biodata and the subject resuscitation strategy to adapt the subject resuscitation treatment.

The resuscitation system uses knowledge of the subject to determine best resuscitation actions to provide a tailored defibrillator resuscitation treatment for the subject. The subject knowledge comprises subject information, e.g. health records, received by the computing device, subject biosignals measured by the sensor and subject biodata measured by the defibrillator.

The computing device may comprise at least one processor, at least one signal transceiver, at least one storage module, at least one display module, at least one user interface. The computing device may comprise any of a smartphone, a tablet, a laptop. The computing device may comprise resuscitation strategy software. The resuscitation strategy software may comprise a software application.

The resuscitation strategy software may create an account for the subject. The resuscitation strategy software may link the subject information to the account. The resuscitation strategy software may link the subject resuscitation strategy to the account. The resuscitation strategy software may link the sensor subject biodata to the account. The resuscitation strategy software may link the defibrillator subject biodata to the account.

The resuscitation strategy software may comprise a resuscitation strategy software algorithm. The resuscitation strategy software algorithm may parse the subject information and use the parsed subject information to create the subject resuscitation strategy. The resuscitation strategy software algorithm may access a database of previously-developed resuscitation strategies categorised by subject information and select a previously-developed resuscitation strategy that best fits the parsed subject information to be the subject resuscitation strategy. The database of previously-developed resuscitation strategies may be stored in the computing device or in a remote location access by the computing device. The subject resuscitation strategy may be output to the defibrillator as a set of software instructions.

The subject information may comprise any of subject health data, subject genetic data, subject lifestyle data, subject demographic data. The subject health data may comprise any of subject medical history, subject cardiac conditions, subject health status, subject illnesses, subject injuries. The subject genetic data may comprise any of genetic test results, genetic variations linked to cardiac conditions, genetic variations linked to musculo-skeletal conditions. The subject lifestyle data may comprise any of sleep data, mood data, stress level data, nutrition data, activity data. The subject demographic data may comprise any of height, weight, gender, ethnicity, location data.

The computing device may input the subject information from any of the subject, a carer of the subject, medical professionals of the subject. The computing device may detect that the subject has been in a healthcare facility and input subject information comprising subject health data updates. The computing device may send a notification to the subject to determine if the subject wants to input the health data updates.

The computing device may receive the sensor subject biosignals and issue a notification to the sensor requesting subject health status data. The computing device may input subject health status data comprising subject health status good data and send a signal to the sensor causing a return to monitoring for a subject cardiac arrest event.

The computing device may treat receipt of the sensor subject biosignals as a subject cardiac arrest event alarm and send a notification to emergency services to alert them of the subject cardiac arrest event. Alternatively, on detection of a subject cardiac arrest event, the sensor may be configured to output a subject cardiac arrest event alarm and sensor subject biosignals to the computing device. The computing device may receive the subject cardiac arrest event alarm and send a notification to emergency services to alert them of the subject cardiac arrest event.

The computing device may be configured to send information about the subject to any of emergency services, a medical facility. The information about the subject may be sent in real-time during resuscitation treatment of the subject.

The sensor may be proximate the subject to detect the subject biosignals. The sensor may be a wearable sensor. The wearable sensor may be a wrist watch capable of detecting biosignals. The wearable sensor may be a chest-based device capable of detecting biosignals.

The sensor may continuously detect subject biosignals. The sensor may periodically detect subject biosignals. The sensor may detect subject biosignals prior to a subject cardiac arrest event and output the prior subject biosignals to the computing device. The subject biosignals detected prior to the subject cardiac arrest event may comprise biosignals detected between the subject cardiac arrest event and any of 2 hours, 1 hour, 45 mins, 30 mins, 15 mins, 10 mins, 5 mins, 3 mins prior to the subject cardiac arrest event.

The sensor may detect subject biosignals comprising any of subject heart rate signals, subject ECG signals, subject blood pressure signals, subject respiration rate signals, subject blood oxygen saturation signals, subject regional blood oxygen saturation signals, photoplethysmography, subject activity data signals, subject position signals, subject location signals. The sensor may detect cessation of subject heart rate signals and use this to determine a subject cardiac arrest event. The sensor may by detect an arrythmia in subject ECG signals and use this to determine a subject cardiac arrest event.

The resuscitation strategy software algorithm of the computing device may use the sensor subject biosignals to adapt the subject resuscitation strategy. The resuscitation strategy software algorithm may access a database of optimal subject biosignals, determine sensor subject biosignals which are suboptimal subject biosignals, and adapt aspects of the subject resuscitation strategy corresponding to the suboptimal subject biosignals.

On determination of a subject cardiac arrest event, the sensor may be configured to repeatedly output sensor subject biosignals to the computing device, and the computing device may be configured to repeatedly use the sensor subject biosignals to adapt the subject resuscitation strategy and output the subject resuscitation strategy to the defibrillator. The computing device may be configured to repeatedly use the sensor subject biosignals and the defibrillator subject biodata to adapt the subject resuscitation strategy and output the subject resuscitation strategy to the defibrillator.

The sensor may further measure subject information about subject lifestyle changes and output the subject information to the computing device. The computing device may use the subject information to adapt the subject resuscitation strategy. The subject lifestyle changes may comprise any of changes in exercise routine, changes in sleep habits, changes in nutritional status.

The defibrillator may measure subject biodata comprising any of subject heart rate biodata, subject ECG biodata, subject ICG biodata, subject blood pressure biodata, subject respiration rate biodata, subject blood oxygen saturation biodata, subject cardiopulmonary resuscitation (CPR) chest compression biodata, a type of the cardiac arrest event, condition of the subject, duration of the cardiac arrest event, subject response to the resuscitation treatment, success of resuscitation treatment.

The defibrillator may comprise a resuscitation treatment algorithm which uses the subject biodata and the subject resuscitation strategy to determine and deliver the subject resuscitation treatment. The subject resuscitation treatment may comprise a set of software instructions which are used by the defibrillator to alter one or more defibrillation shock waveform characteristics for the subject. The defibrillation shock waveform characteristics may comprise any of pulse duration, energy, voltage, tilt, interphase spacing. The subject resuscitation treatment may comprise a set of software instructions which are used by the defibrillator to alter feedback presented to a rescuer using the defibrillator to deliver the resuscitation treatment for the subject. The feedback may comprise any of perform hands-only CPR, perform mouth to mouth CPR, adjust CPR chest compression duration, adjust CPR chest compression rate, adjust CPR chest compression depth, adjust CPR chest compression recoil. The feedback may comprise prompts to the rescuer comprising any of audio prompts, visual prompts, haptic prompts, other sensory prompts.

The resuscitation strategy software algorithm of the computing device may use the defibrillator subject biodata to adapt the subject resuscitation strategy. The resuscitation strategy software algorithm may access a database of optimal subject biodata, determine defibrillator subject biodata which are suboptimal subject biodata, and adapt aspects of the subject resuscitation strategy corresponding to the suboptimal subject biodata.

The defibrillator may comprise a receiver configured to receive subject information from the rescuer. The subject information from the rescuer may comprise any of subject gender, subject ethnicity, subject height, subject weight. The defibrillator may output the subject information to the computing device. The computing device may use the subject information to adapt the subject resuscitation strategy and output the subject resuscitation strategy to the defibrillator.

The resuscitation system may be deployed in the subject's home using a home defibrillator. The resuscitation system may be deployed outside of the subject's home using a public defibrillator. The defibrillator may be an automated external defibrillator (AED).

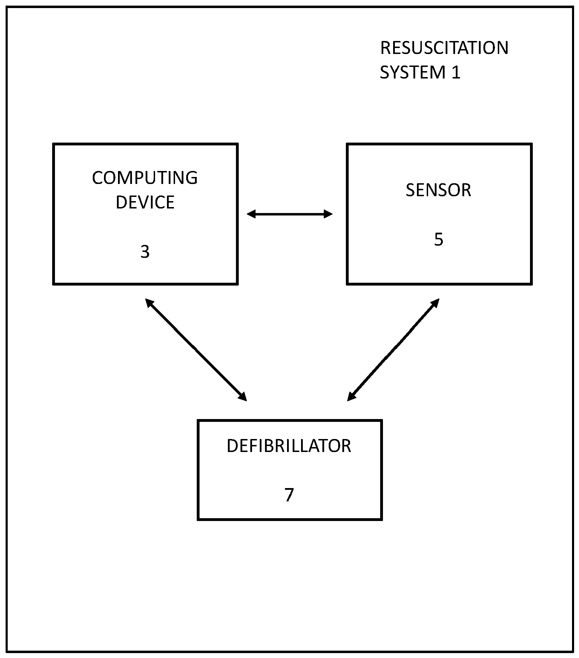

An embodiment of the invention will now be described, by way of example only, with reference to the accompanying drawing, which is a schematic representation of a resuscitation system.

Referring to the drawing, a resuscitation system 1 for use in the resuscitation of a subject, comprises a computing device 3, a sensor 5 and a defibrillator 7. The computing device 3, sensor 5 and defibrillator 7 are connected to each other, preferably wirelessly, but it will be appreciated that, in some circumstances, wired connections could be used.

In this embodiment, the computing device 3 comprises a smartphone. The smartphone comprises at least one processor, at least one signal transceiver, a resuscitation strategy software in the form of a software application, at least one storage module, at least one display module and at least one user interface.

On receipt of the resuscitation system 1, the resuscitation strategy software of the computing device 3 is used to create an account for the subject on the computing device 3. The sensor 5 and the defibrillator are linked to the subject account. When subject information is received by the computing device 3, this is linked to the subject account. When the subject resuscitation strategy is created, this is also linked to the subject account.

The computing device 3 inputs subject information and uses the subject information to create a subject resuscitation strategy. The resuscitation strategy software comprises a resuscitation strategy software algorithm which parses the subject information and uses the parsed subject information to create the subject resuscitation strategy. The resuscitation strategy software algorithm accesses a database of previously-developed resuscitation strategies categorised by subject information and selects a previously-developed resuscitation strategy that best fits the parsed subject information to be the subject resuscitation strategy. The database of previously-developed resuscitation strategies may be stored in the computing device or in a remote location access by the computing device. The subject resuscitation strategy is output to the defibrillator as a set of software instructions.

The subject information input by the computing device 3 comprises any of subject health data, subject genetic data, subject lifestyle data, subject demographic data. The subject health data may comprise any of subject medical history, subject cardiac conditions, subject health status, subject illnesses, subject injuries. The subject genetic data may comprise any of genetic test results, genetic variations linked to cardiac conditions, genetic variations linked to musculo-skeletal conditions. The subject lifestyle data may comprise any of sleep data, mood data, stress level data, nutrition data, activity data. The subject demographic data may comprise any of height, weight, gender, ethnicity, location data.

The subject information may be input by the computing device 3 from any of the subject, a carer of the subject, medical professionals of the subject. The computing device 3 may detect that the subject has been in a healthcare facility and input subject information comprising subject health data updates. The computing device 3 may send a notification to the subject to determine if the subject wants to input the health data updates.

In this embodiment, the sensor 5 is a wearable sensor, such as a wrist watch or a chest-based sensor. The sensor 5 is configured to detect subject biosignals and use the subject biosignals to monitor for a subject cardiac arrest event. The sensor 5 may continuously or periodically detect subject biosignals. The sensor 5 may detect subject biosignals prior to a subject cardiac arrest event. The subject biosignals detected prior to the subject cardiac arrest event may comprise biosignals detected between the subject cardiac arrest event and any of 2 hours, 1 hour, 45 mins, 30 mins, 15 mins, 10 mins, 5 mins, 3 mins prior to the subject cardiac arrest event.

The sensor 5 detects subject biosignals comprising any of subject heart rate signals, subject ECG signals, subject blood pressure signals, subject respiration rate signals, subject blood oxygen saturation signals, subject activity data signals, subject position signals, subject location signals. The sensor 5 detects cessation of subject heart rate signals and uses this to determine a subject cardiac arrest event. The sensor 5 detects an arrythmia in subject ECG signals and uses this to determine a subject cardiac arrest event.

On determination of a subject cardiac arrest event, the sensor 5 is configured to output sensor subject biosignals to the computing device 3. The subject biosignals may be biosignals detected prior to a subject cardiac arrest event.

The computing device 5 treats receipt of the sensor subject biosignals as a subject cardiac arrest event alarm and sends a notification to emergency services to alert them of the subject cardiac arrest event. The resuscitation strategy software of the computing device 3 links the sensor subject biodata to the subject account.

The computing device 3 is configured to use the sensor subject biosignals to adapt the subject resuscitation strategy and output the subject resuscitation strategy to the defibrillator 7. The resuscitation strategy software algorithm of the computing device 3 uses the sensor subject biosignals to adapt the subject resuscitation strategy. The resuscitation strategy software algorithm accesses a database of optimal subject biosignals, determines sensor subject biosignals which are suboptimal subject biosignals, and adapts aspects of the subject resuscitation strategy corresponding to the suboptimal subject biosignals. For example, the resuscitation strategy software algorithm may determine sensor subject biosignals relating to the subject heart rate are suboptimal and adapt an aspect of the subject resuscitation strategy relating to rate of CPR to be delivered to the subject.

In this embodiment, the defibrillator 7 is an AED and the resuscitation system 1 is deployed in the subject's home using a home AED.

The defibrillator 7 is configured to measure subject biodata, use the subject biodata and the subject resuscitation strategy received from the computing device 3 to determine and deliver a subject resuscitation treatment. The defibrillator 7 measures subject biodata comprising any of subject heart rate biodata, subject ECG biodata, subject ICG biodata, subject blood pressure biodata, subject respiration rate biodata, subject blood oxygen saturation biodata, subject cardiopulmonary resuscitation (CPR) chest compression biodata. The defibrillator 7 further measures subject biodata comprising any of a type of the cardiac arrest event, condition of the subject, duration of the cardiac arrest event, subject response to the resuscitation treatment, success of resuscitation treatment.

The defibrillator 7 comprises a resuscitation treatment algorithm which uses the subject biodata and the subject resuscitation strategy to determine and deliver the subject resuscitation treatment. The subject resuscitation treatment comprises a set of software instructions which are used by the defibrillator to alter one or more defibrillation shock waveform characteristics for the subject. The defibrillation shock waveform characteristics may comprise any of pulse duration, energy, voltage, tilt, interphase spacing. The subject resuscitation treatment further comprises a set of software instructions which are used by the defibrillator to alter feedback presented to a rescuer using the defibrillator to deliver the resuscitation treatment for the subject. The feedback may comprise any of perform hands-only CPR, perform mouth to mouth CPR, adjust CPR chest compression duration, adjust CPR chest compression rate, adjust CPR chest compression depth, adjust CPR chest compression recoil. The feedback may comprise prompts to the rescuer comprising any of audio prompts, visual prompts, haptic prompts, other sensory prompts.

During subject resuscitation treatment delivered by the defibrillator 7, the defibrillator 7 is configured to repeatedly measure subject biodata and output defibrillator subject biodata to the computing device 3. The resuscitation strategy software of the computing device 3 links the defibrillator subject biodata to the subject account. The computing device 3 is configured to repeatedly use the defibrillator subject biodata to adapt the subject resuscitation strategy. The resuscitation strategy software algorithm of the software of the computing device 3 accesses a database of optimal subject biodata, determines defibrillator subject biodata which are suboptimal subject biodata, and adapts aspects of the subject resuscitation strategy corresponding to the suboptimal subject biodata. For example, the resuscitation strategy software algorithm may determine defibrillator subject biodata relating to the subject ECG are suboptimal and adapt an aspect of the subject resuscitation strategy relating to a tilt characteristic of a defibrillation shock waveform to be delivered to the subject. The computing device 3 outputs the subject resuscitation strategy to the defibrillator 7, which uses the measured subject biodata and the subject resuscitation strategy to adapt the subject resuscitation treatment. This process is carried out repeatedly during subject resuscitation treatment, for example, the defibrillator may output defibrillator subject biodata to the computing device 3 every 5 seconds.

The defibrillator 7 may comprise a receiver (not shown) configured to receive subject information from the rescuer, such as subject gender, subject ethnicity, subject height, subject weight. The defibrillator 7 may output the subject information to the computing device 3 and the computing device 3 may use the subject information to adapt the subject resuscitation strategy and output the subject resuscitation strategy to the defibrillator 7.

The computing device 3 is configured to send information about the subject to any of emergency services, a medical facility, in real-time, during subject resuscitation treatment.

## Claims

1. A resuscitation system (1) for use in the resuscitation of a subject, comprising:
a computing device (3) configured to input subject information and use the subject information to create a subject resuscitation strategy,
a sensor (5) configured to detect subject biosignals and use the subject biosignals to monitor for a subject cardiac arrest event, and
a defibrillator (7) configured to determine and deliver a subject resuscitation treatment, wherein
on determination of a subject cardiac arrest event, the sensor (5) is configured to output sensor subject biosignals to the computing device (3),
the computing device (3) is configured to use the sensor subject biosignals to adapt the subject resuscitation strategy and output the subject resuscitation strategy to the defibrillator (7),
the defibrillator (7) is configured to measure subject biodata, use the subject biodata and the subject resuscitation strategy to determine and deliver a subject resuscitation treatment, and
during subject resuscitation treatment,
the defibrillator (7) is configured to repeatedly measure subject biodata and output defibrillator subject biodata to the computing device (3),
the computing device (3) is configured to repeatedly use the defibrillator subject biodata to adapt the subject resuscitation strategy and output the subject resuscitation strategy to the defibrillator (7),
the defibrillator (7) is configured to repeatedly use the measured subject biodata and the subject resuscitation strategy to adapt the subject resuscitation treatment.

2. A resuscitation system (1) according to claim 1 in which the computing device (3) comprises a resuscitation strategy software algorithm which parses the subject information and uses the parsed subject information to create the subject resuscitation strategy.

3. A resuscitation system (1) according to claim 2 in which the resuscitation strategy software algorithm accesses a database of previously-developed resuscitation strategies categorised by subject information and selects a previously-developed resuscitation strategy that best fits the parsed subject information to be the subject resuscitation strategy.

4. A resuscitation system (1) according to any preceding claim in which the computing device (3) comprises a resuscitation strategy software algorithm which uses the sensor subject biosignals to adapt the subject resuscitation strategy.

5. A resuscitation system (1) according to claim 4 in which the resuscitation strategy software algorithm accesses a database of optimal subject biosignals, determines sensor subject biosignals which are suboptimal subject biosignals, and adapts aspects of the subject resuscitation strategy corresponding to the suboptimal subject biosignals.

6. A resuscitation system (1) according to any preceding claim in which the computing device (3) comprises a resuscitation strategy software algorithm which uses the defibrillator subject biodata to adapt the subject resuscitation strategy.

7. A resuscitation system (1) according to claim 6 in which the resuscitation strategy software algorithm accesses a database of optimal subject biodata, determines defibrillator subject biodata which are suboptimal subject biodata, and adapts aspects of the subject resuscitation strategy corresponding to the suboptimal subject biodata.

8. A resuscitation system (1) according to any preceding claim in which the subject information comprises any of subject health data, subject genetic data, subject lifestyle data, subject demographic data.

9. A resuscitation system (1) according to any preceding claim in which the sensor (5) detects subject biosignals prior to a subject cardiac arrest event and outputs the prior subject biosignals to the computing device (3).

10. A resuscitation system (1) according to any preceding claim in which the sensor (5) detects subject biosignals comprising any of subject heart rate signals, subject ECG signals, subject blood pressure signals, subject respiration rate signals, subject blood oxygen saturation signals, subject activity data signals, subject position signals, subject location signals.

11. A resuscitation system (1) according to any preceding claim in which the sensor (5) further measures subject information about subject lifestyle changes and outputs the subject information to the computing device (3), which uses the subject information to adapt the subject resuscitation strategy.

12. A resuscitation system (1) according to any preceding claim in which the defibrillator (7) measures subject biodata comprising any of subject heart rate biodata, subject ECG biodata, subject ICG biodata, subject blood pressure biodata, subject respiration rate biodata, subject blood oxygen saturation biodata, subject cardiopulmonary resuscitation (CPR) chest compression biodata, a type of the cardiac arrest event, condition of the subject, duration of the cardiac arrest event, subject response to the resuscitation treatment, success of resuscitation treatment.

13. A resuscitation system (1) according to any preceding claim in which the subject resuscitation treatment comprises a set of software instructions which are used by the defibrillator (7) to alter one or more defibrillation shock waveform characteristics for the subject.

14. A resuscitation system (1) according to claim 13 in which the defibrillation shock waveform characteristics comprise any of pulse duration, energy, voltage, tilt, interphase spacing.

15. A resuscitation system (1) according to any preceding claim in which the subject resuscitation treatment comprises a set of software instructions which are used by the defibrillator (7) to alter feedback presented to a rescuer using the defibrillator to deliver the resuscitation treatment for the subject.

16. A resuscitation system (1) according to claim 15 in which the feedback comprises any of perform hands-only CPR, perform mouth to mouth CPR, adjust CPR chest compression duration, adjust CPR chest compression rate, adjust CPR chest compression depth, adjust CPR chest compression recoil.

## Patentansprüche

1. Reanimationssystem (1) zur Verwendung bei der Reanimation eines Subjekts, umfassend:
eine Rechenvorrichtung (3), die dazu konfiguriert ist, Subjektinformationen einzugeben und die Subjektinformationen zum Erstellen einer Reanimationsstrategie für das Subjekt zu verwenden,
einen Sensor (5), der dazu konfiguriert ist, Biosignale des Subjekts zu erkennen und die Biosignale des Subjekts zum Überwachen eines Herzstillstandsereignisses des Subjekts zu verwenden, und
einen Defibrillator (7), der dazu konfiguriert ist, eine Reanimationsbehandlung des Subjekts zu bestimmen und abzugeben, wobei
bei Bestimmung eines Herzstillstandsereignisses des Subjekts der Sensor (5) dazu konfiguriert ist, Sensor-Biosignale des Subjekts an die Rechenvorrichtung (3) auszugeben,
die Rechenvorrichtung (3) dazu konfiguriert ist, die Sensor-Biosignale des Subjekts zu verwenden, um die Reanimationsstrategie für das Subjekt anzupassen und die Reanimationsstrategie des Subjekts an den Defibrillator (7) auszugeben,
der Defibrillator (7) dazu konfiguriert ist, Biodaten des Subjekts zu messen, die Biodaten des Subjekts und die Reanimationsstrategie für das Subjekt zu verwenden, um eine Reanimationsbehandlung des Subjekts zu bestimmen und abzugeben, und
während der Reanimationsbehandlung des Subjekts,
der Defibrillator (7) dazu konfiguriert ist, wiederholt Biodaten des Subjekts zu messen und die Defibrillator-Biodaten des Subjekts an die Rechenvorrichtung (3) auszugeben,
die Rechenvorrichtung (3) dazu konfiguriert ist, die Defibrillator-Biodaten des Subjekts wiederholt zu verwenden, um die Reanimationsstrategie für das Subjekt anzupassen und die Reanimationsstrategie für das Subjekt an den Defibrillator (7) auszugeben,
der Defibrillator (7) dazu konfiguriert ist, die gemessenen Biodaten des Subjekts und die Reanimationsstrategie für das Subjekt wiederholt zu verwenden, um die Reanimationsbehandlung des Subjekts anzupassen.

2. Reanimationssystem (1) nach Anspruch 1, bei dem die Rechenvorrichtung (3) einen Softwarealgorithmus für die Reanimationsstrategie umfasst, der die Subjektinformationen analysiert und die analysierten Subjektinformationen zum Erstellen der Reanimationsstrategie für das Subjekt verwendet.

3. Reanimationssystem (1) nach Anspruch 2, bei dem der Softwarealgorithmus für die Reanimationsstrategie auf eine Datenbank mit zuvor entwickelten, nach Subjektinformationen kategorisierten Reanimationsstrategien zugreift und eine zuvor entwickelte Reanimationsstrategie, die am besten zu den analysierten Subjektinformationen passt, als die Reanimationsstrategie für das Subjekt auswählt.

4. Reanimationssystem (1) nach einem vorhergehenden Anspruch, bei dem die Rechenvorrichtung (3) einen Softwarealgorithmus für die Reanimationsstrategie umfasst, der die Sensor-Biosignale des Subjekts verwendet, um die Reanimationsstrategie für das Subjekt anzupassen.

5. Reanimationssystem (1) nach Anspruch 4, bei dem der Softwarealgorithmus der Reanimationsstrategie auf eine Datenbank mit optimalen Biosignalen des Subjekts zugreift, Sensor-Biosignale des Subjekts bestimmt, die suboptimale Biosignale des Subjekts sind, und Aspekte der Reanimationsstrategie für das Subjekt entsprechend den suboptimalen Biosignalen des Subjekts anpasst.

6. Reanimationssystem (1) nach einem vorhergehenden Anspruch, bei dem die Rechenvorrichtung (3) einen Softwarealgorithmus für die Reanimationsstrategie umfasst, der die Defibrillator-Biodaten des Subjekts verwendet, um die Reanimationsstrategie für das Subjekt anzupassen.

7. Reanimationssystem (1) nach Anspruch 6, bei dem der Softwarealgorithmus der Reanimationsstrategie auf eine Datenbank mit optimalen Biodaten des Subjekts zugreift, Defibrillator-Biodaten des Subjekts bestimmt, die suboptimale Biodaten des Subjekts sind, und Aspekte der Reanimationsstrategie für das Subjekt entsprechend den suboptimalen Biodaten des Subjekts anpasst.

8. Reanimationssystem (1) nach einem vorhergehenden Anspruch, bei dem die Subjektinformationen Beliebige von Gesundheitsdaten des Subjekts, genetischen Daten des Subjekts, Daten zum Lebensstil des Subjekts, demografischen Daten des Subjekts umfassen.

9. Reanimationssystem (1) nach einem vorhergehenden Anspruch, bei dem der Sensor (5) Biosignale des Subjekts vor einem Herzstillstandsereignis des Subjekts erkennt und die vorherigen Biosignale des Subjekts an die Rechenvorrichtung (3) ausgibt.

10. Reanimationssystem (1) nach einem vorhergehenden Anspruch, bei dem der Sensor (5) Biosignale des Subjekts erkennt, die Beliebige von Herzfrequenzsignalen des Subjekts, EKG-Signalen des Subjekts, Blutdrucksignalen des Subjekts, Atemfrequenzsignalen des Subjekts, Signalen einer Sauerstoffsättigung im Blut des Subjekts, Aktivitätsdatensignalen des Subjekts, Positionssignalen des Subjekts, Standortsignalen des Subjekts umfassen.

11. Reanimationssystem (1) nach einem vorhergehenden Anspruch, bei dem der Sensor (5) ferner Subjektinformationen über Lebensstiländerungen des Subjekts misst und die Subjektinformationen an die Rechenvorrichtung (3) ausgibt, die die Subjektinformationen verwendet, um die Reanimationsstrategie für das Subjekt anzupassen.

12. Reanimationssystem (1) nach einem vorhergehenden Anspruch, bei dem der Defibrillator (7) Biodaten des Subjekts misst, die Beliebige von Herzfrequenzbiodaten des Subjekts, EKG-Biodaten des Subjekts, IKG-Biodaten des Subjekts, Blutdruckbiodaten des Subjekts, Atemfrequenzbiodaten des Subjekts, Biodaten zur Sauerstoffsättigung im Blut des Subjekts, Biodaten zur Thoraxkompression der Herz-Lungen-Reanimation (CPR) des Subjekts, einer Art des Herzstillstandsereignisses, einem Zustand des Subjekts, einer Dauer des Herzstillstandsereignisses, einem Ansprechen des Subjekts auf die Reanimationsbehandlung, einem Erfolg der Reanimationsbehandlung umfassen.

13. Reanimationssystem (1) nach einem vorhergehenden Anspruch, bei dem die Reanimationsbehandlung des Subjekts einen Satz von Software-Anweisungen umfasst, die durch den Defibrillator (7) verwendet werden, um eine oder mehrere Eigenschaften der Defibrillationsstoßwellenform für das Subjekts zu verändern.

14. Reanimationssystem (1) nach Anspruch 13, bei dem die Eigenschaften der Defibrillationsstoßwellenform Beliebige von Impulsdauer, Energie, Spannung, Neigung, Phasenabstand umfassen.

15. Reanimationssystem (1) nach einem vorhergehenden Anspruch, bei dem die Reanimationsbehandlung des Subjekts einen Satz von Software-Anweisungen umfasst, die durch den Defibrillator (7) verwendet werden, um eine Rückmeldung zu verändern, die einem Helfer, der den Defibrillator verwendet, um die Reanimationsbehandlung für das Subjekt abzugeben, präsentiert wird.

16. Reanimationssystem (1) nach Anspruch 15, bei dem die Rückmeldung Beliebige von Durchführen einer CPR nur mit den Händen, Durchführen einer Mund-zu-Mund-CPR, Anpassen der CPR-Thoraxkompressionsdauer, Anpassen der CPR-Thoraxkompressionsfrequenz, Anpassen der CPR-Thoraxkompressionstiefe, Anpassen der CPR-Thoraxkompressionsentlastung umfasst.

## Revendications

1. Système de réanimation (1) destiné à être utilisé pour la réanimation d'un sujet, comprenant :
un dispositif informatique (3) configuré pour entrer des informations sur le sujet et utiliser les informations sur le sujet pour créer une stratégie de réanimation du sujet,
un capteur (5) configuré pour détecter les biosignaux du sujet et utiliser les biosignaux du sujet pour surveiller un événement d'arrêt cardiaque du sujet, et
un défibrillateur (7) configuré pour déterminer et administrer un traitement de réanimation à un sujet,
lors de la détermination d'un événement d'arrêt cardiaque d'un sujet, ledit capteur (5) étant configuré pour transmettre des biosignaux du sujet provenant du capteur au dispositif informatique (3),
ledit dispositif informatique (3) étant configuré pour utiliser les biosignaux du sujet provenant du capteur de manière à adapter la stratégie de réanimation du sujet et transmettre la stratégie de réanimation du sujet au défibrillateur (7),
ledit défibrillateur (7) étant configuré pour mesurer les biodonnées du sujet, utiliser les biodonnées du sujet et la stratégie de réanimation du sujet pour déterminer et administrer un traitement de réanimation du sujet, et
pendant le traitement de réanimation du sujet,
ledit défibrillateur (7) étant configuré pour mesurer de manière répétée les biodonnées du sujet et transmettre les biodonnées du sujet provenant du défibrillateur au dispositif informatique (3),
ledit dispositif informatique (3) étant configuré pour utiliser de manière répétée les biodonnées du sujet provenant du défibrillateur de manière à adapter la stratégie de réanimation du sujet et transmettre la stratégie de réanimation du sujet au défibrillateur (7),
ledit défibrillateur (7) étant configuré pour utiliser de manière répétée les biodonnées du sujet mesurées et la stratégie de réanimation du sujet de manière à adapter le traitement de réanimation du sujet.

2. Système de réanimation (1) selon la revendication 1, ledit dispositif informatique (3) comprenant un algorithme logiciel de stratégie de réanimation qui analyse les informations sur le sujet et utilise les informations sur le sujet analysées pour créer la stratégie de réanimation du sujet.

3. Système de réanimation (1) selon la revendication 2, ledit algorithme logiciel de stratégie de réanimation accédant à une base de données de stratégies de réanimation précédemment développées, classées par informations sur le sujet et sélectionnant une stratégie de réanimation développée précédemment qui correspond le mieux aux informations sur le sujet analysées pour qu'elle devienne la stratégie de réanimation du sujet.

4. Système de réanimation (1) selon l'une quelconque des revendications précédentes, ledit dispositif informatique (3) comprenant un algorithme logiciel de stratégie de réanimation qui utilise les biosignaux du sujet détectés par le capteur de manière à adapter la stratégie de réanimation du sujet.

5. Système de réanimation (1) selon la revendication 4, ledit algorithme logiciel de stratégie de réanimation accédant à une base de données de biosignaux optimaux du sujet, déterminant les biosignaux du sujet détectés par le capteur qui sont des biosignaux sous-optimaux du sujet, et adaptant les aspects de la stratégie de réanimation du sujet correspondant aux biosignaux sous-optimaux du sujet.

6. Système de réanimation (1) selon l'une quelconque des revendications précédentes, ledit dispositif informatique (3) comprenant un algorithme logiciel de stratégie de réanimation qui utilise les biodonnées du sujet provenant du défibrillateur de manière à adapter la stratégie de réanimation du sujet.

7. Système de réanimation (1) selon la revendication 6, ledit algorithme logiciel de stratégie de réanimation accédant à une base de données de biodonnées optimales du sujet, déterminant les biodonnées du sujet provenant du défibrillateur qui sont des biodonnées sous-optimales du sujet, et adaptant les aspects de la stratégie de réanimation du sujet correspondant aux biodonnées sous-optimales du sujet.

8. Système de réanimation (1) selon l'une quelconque des revendications précédentes, lesdites informations sur le sujet comprenant l'une quelconque des données de santé du sujet, des données génétiques du sujet, des données sur le style de vie du sujet et des données démographiques du sujet.

9. Système de réanimation (1) selon l'une quelconque des revendications précédentes, ledit capteur (5) détectant les biosignaux du sujet avant un événement d'arrêt cardiaque du sujet et transmettant les biosignaux du sujet antérieurs au dispositif informatique (3).

10. Système de réanimation (1) selon l'une quelconque des revendications précédentes, ledit capteur (5) détectant des biosignaux du sujet comprenant l'un quelconque des signaux de fréquence cardiaque du sujet, des signaux d'ECG du sujet, des signaux de tension artérielle du sujet, des signaux de fréquence respiratoire du sujet, des signaux de saturation en oxygène du sang du sujet, des signaux de données d'activité, des signaux de position du sujet, des signaux de localisation du sujet.

11. Système de réanimation (1) selon l'une quelconque des revendications précédentes, ledit capteur (5) mesurant en outre des informations sur le sujet concernant les changements de style de vie du sujet et transmettant les informations sur le sujet au dispositif informatique (3), qui utilise les informations sur le sujet de manière à adapter la stratégie de réanimation du sujet.

12. Système de réanimation (1) selon l'une quelconque des revendications précédentes, ledit défibrillateur (7) mesurant les biodonnées du sujet comprenant une quelconque biodonnée parmi les biodonnées de fréquence cardiaque du sujet, les biodonnées d'ECG du sujet, les biodonnées d'ICG du sujet, les biodonnées de tension artérielle du sujet, les biodonnées de fréquence respiratoire du sujet, les biodonnées de saturation en oxygène du sang du sujet, les biodonnées de compression thoracique du sujet en réanimation cardio-pulmonaire (RCP), un type d'événement d'arrêt cardiaque, l'état du sujet, la durée de l'événement d'arrêt cardiaque, la réponse du sujet au traitement de réanimation, le succès du traitement de réanimation.

13. Système de réanimation (1) selon l'une quelconque des revendications précédentes, ledit traitement de réanimation du sujet comprenant un ensemble d'instructions logicielles qui sont utilisées par le défibrillateur (7) pour modifier une ou plusieurs caractéristiques de forme d'onde de choc de défibrillation pour le sujet.

14. Système de réanimation (1) selon la revendication 13, lesdites caractéristiques de forme d'onde de choc de défibrillation comprenant une quelconque caractéristique parmi la durée d'impulsion, l'énergie, la tension, l'inclinaison et l'espacement interphase.

15. Système de réanimation (1) selon l'une quelconque des revendications précédentes, ledit traitement de réanimation du sujet comprenant un ensemble d'instructions logicielles qui sont utilisées par le défibrillateur (7) pour modifier la rétroaction présentée à un secouriste utilisant le défibrillateur afin d'administrer le traitement de réanimation au sujet.

16. Système de réanimation (1) selon la revendication 15, ladite rétroaction comprenant une quelconque action parmi : effectuer une RCP avec les mains uniquement, effectuer une RCP avec bouche à bouche, ajuster la durée de la compression thoracique de la RCP, ajuster le taux de compression thoracique de la RCP, ajuster la profondeur de la compression thoracique de la RCP, ajuster la détente de la compression thoracique de la RCP.
